(19) Europäisches Patentamt
European Patent Office
Office européen des brevets

(11) Publication number: 0 509 180 A1

# (12) EUROPEAN PATENT APPLICATION

(21) Application number: **91401036.8**

(22) Date of filing: **18.04.91**

(43) Date of publication of application:
**21.10.92 Bulletin 92/43**

(84) Designated Contracting States:
**FR**

(51) Int. Cl.$^5$: **A61K 31/195**

(71) Applicant: **MERRELL DOW PHARMACEUTICALS INC.**
**2110 East Galbraith Road**
**Cincinnati Ohio 45215-6300(US)**

(72) Inventor: **Seiler, Nikolaus**
**1 Rue Jean-Sebastien Bach**
**F-67150 Matzenheim(FR)**

(74) Representative: **Gillard, Marie-Louise et al**
**Cabinet Beau de Loménie 55, Rue d'Amsterdam**
**F-75008 Paris(FR)**

## (54) Use of GABA-T inhibitors for the treatment of schizophrenia and phencyclidine intoxication.

(57) This invention relates to the use of gamma-aminobutyric acid transaminase inhibitors in the treatment of phencyclidine intoxication and in the treatment of schizophrenia.

EP 0 509 180 A1

This invention relates to the use of gamma-aminobutyric acid transaminase inhibitors in the treatment of phencyclidine intoxication and in the treatment of schizophrenia.

Phencyclidine (i.e., 1-(1-phenylcyclohexylpiperidine)) is an arylcycloalkylamine of the formula

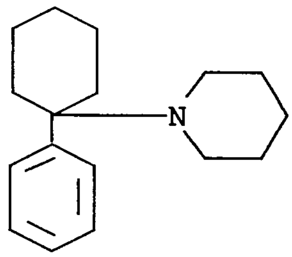

The compound was first developed as an anesthetic for animals and then as a general anesthetic in man. However, its use as a general anesthetic was stopped because patients experienced delirium after they emerged from anesthesia. Its use as a veterinary anesthetic has also been discontinued.

Phencyclidine and its related arylcycloalkylamine congeners have been described as dissociative anesthetics as they have hallucinogenic, analgesic and anesthetic properties. Phencyclidine, and its related congeners, can cause acute behavioral toxicity such as intoxication, aggression, brief confusional states, coma, convulsions and psychotic states. Patients with schizophrenia or with schizophrenic tendencies being particularly vulnerable to the psychogenic actions of phencyclidine.

Unfortunately, phencyclidine (and its congeners) has found a use as a street drug and, as such, is often referred to under names as PCP, Angel Dust, crystal, horse tranquilizer and peace pill. In its street drug use, it often leads to the side effect known as phencyclidine intoxication, generally manifested by a staggering gait, slurred speech, nystagmus, blank stares and in many instances leads to hospital admissions for schizophrenia-like psychoses. In practice such patients may be erroneously confined to long stays of hospitalization and be treated as a schizophrenic. Thus, this invention has as one of its objects the treatment of phencyclidine intoxication *per se*, and to obviate hospital time and expense when treating patients for schizophrenia before the attending physician recognizes that the patient had been suffering from phencyclidine intoxication. Another object of this invention is to treat hallucinatory endogenous psychoses of the type manifested in patients suffering from schizophrenia.

The foregoing objectives may be achieved by increasing brain GABA concentrations by the administration of a therapeutically effective amount of a gamma-aminobutyric acid transaminase inhibitor, said inhibitor preferably being in the form of a pharmaceutically acceptable salt thereof.

In determining efficacy for the above-stated uses of gamma-aminobutyric acid transaminase inhibitors standard laboratory models are employed wherein effects on phencyclidine-induced locomotor activity are determined. One such test employed is: male CDI albino mice (weighing 30-40 grams) (Charles River, St. Aubin-les-Elbeuf, France) kept under standardized conditions (22°C, 60% relative humidity; 12 hours light - 12 hours dark cycle; water and standard diet *ad libitum*). Locomotor activity is determined using the Event-Counter of Columbus Instr. Corp. (Columbus, Ohio). The animals (six per cage) are brought into a room which is equipped with an actimeter, and allowed to adapt to the new environment for several hours. Phencyclidine is given 5 hours after administration of the GABA-T inhibitors. The dose of phencyclidine used is 5 μg/g in 10 μl of physiological saline, i.p. Immediately after phencyclidine administration (or of the same volume of physiological saline in the case of controls), the animals are placed individually into standard plastic cages (23 cm × 17 cm), and the actimeter is set into action. Total time of observation: 35 minutes. The scores of 5 minute intervals are integrated and stored by the computer.

In order to test "direct" effects of the drugs, locomotor activity was determined, starting 10 minutes after intraperitoneal administration of 150 mg/kg (4S)-allenylGABA, and 400 mg/kg vigabatrin, respectively.

As shown from the results in Table I, neither spontaneous, nor phencyclidine-induced locomotor behavior was significantly affected by this treatment, indicating that the GABA-T inhibitors had no direct action on locomotor behavior.

The locomotor behavior of mice with elevated brain GABA concentrations was tested as follows: vigabatrin and (4S)-allenylGABA were administered intraperitoneally in a dose-range from 100-800 mg/kg and 50-300 mg/kg, respectively. Within this dose range whole brain GABA concentrations, as observed 5 hours post injection were between 2.5 and 10 μmol/g.

Spontaneous locomotor activity was not significantly affected by vigabatrin, although there was a tendency towards lower values with increasing dose (Table II). In phencyclidine-treated mice, a significant

decrease of locomotor activity by vigabatrin was noticed in all animals receiving a dose of 200-700 mg/kg (Table II). The lowest value (observed after administration of 400 mg/kg vigabatrin) was 55% of that of phencyclidine-treated controls. Increase of the dose of vigabatrin above 400 mg/kg caused a tendency to increase rather than to decrease locomotor activity.

As appears from the results in Table III, administration of (4S)-allenylGABA in a dose range from 50 to 300 mg/kg did not affect spontaneous locomotor activity of mice. At the very high dose of 600 mg/kg the animals were sedated, and revealed little motor activity (not shown). In phencyclidine-treated hyperactive animals, a dose-related decrease of locomotor activity could be observed. At the highest dose given (300 mg/kg) phencyclidine-induced hypermotility was reduced by 75%. A statistically significant difference between (4S)-allenylGABA-pretreated mice, and mice receiving only phencyclidine, was seen at a dose of ≧ 100 mg/kg (4S)-allenylGABA.

TABLE I. **Effect of administration of vigabatrin and (4S)-allenylGABA on spontaneous and PCP-induced locomotor activity of mice**

Mean scores ± S.D. (number of animals in parentheses)

| Drug | Dose mg/kg i.p. | Spontaneous locomotor activity | PCP-induced locomotor activity |
|---|---|---|---|
| None | - | 339 ± 235 (11) | 2730 ± 1270 (11) |
| Vigabatrin | 400 | 528 ± 239 ( 6) | 2460 ± 1220 (11) |
| (4S)-allenylGABA | 150 | 688 ± 308 ( 6) | 2680 ± 1040 (11) |

Vigabatrin and (4S)-allenylGABA were given 10 minutes before phencyclidine (5 mg/kg), and physiol. saline, respectively.

TABLE II. **Effect of pretreatment with vigabatrin on spontaneous and PCP-induced locomotor activity of mice**

Mean scores ± S.D. (number of animals in parentheses)

| Vigabatrin Dose mg/kg | Spontaneous locomotor activity | PCP-induced locomotor activity |
|---|---|---|
| 0 | 597 ± 348 (24) | 3080 ± 1200 (18) |
| 100 | - | 2360 ± 1100 (12) |
| 200 | 505 ± 344 (6) | 1970 ± 730 (12)* |
| 400 | 471 ± 306 (12) | 1690 ± 1385 (12)* |
| 600 | 361 ± 139 (6) | 2180 ± 410 (12)* |
| 700 | 441 ± 298 (6) | 1780 ± 1080 (12) |
| 800 | 435 ± 275 (6) | 2100 ± 1780 (12)* |

Vigabatrin was given i.p. 5 h before phencyclidine (5 mg/kg). The asterisk indicates a statistically significant difference ($p \leq 0.05$) between controls and vigabatrin-treated mice. (Analysis of variance for single factor experiments.)

TABLE III. **Effect of pretreatment with (4S)-allenylGABA on spontaneous and PCP-induced locomotor activity of mice**

Mean scores ± S.D. (number of animals in parentheses)

| (4S)-allenylGABA Dose mg/kg | Spontaneous locomotor activity | PCP-induced locomotor activity |
|---|---|---|
| 0 | 472.303 (18) | 3610.1400 (18) |
| 50 | 306.244 ( 6) | 2760.1420 (12) |
| 100 | 406.221 ( 6) | 1770.775 (12)* |
| 150 | 315.304 ( 6) | 1800.1370 (6)* |
| 300 | 445.420 ( 6) | 880.1000 (12)* |

(4S)-allenylGABA was given i.p. 5 h before phencyclidine (5 mg/kg). The asterisk indicates a statistically significant difference ($p \leq 0.01$) between controls and (4S)-allenylGABA-treated mice. (Analysis of variance for single factor experiments.)

The effects of vigabatrin, (i.e., (R)S-4-aminohex-5-enoic acid) and (4S)-allenylGABA, (i.e., (S)-4-allenyl-GABA; (S)-4-amino-5,6-heptadienoic acid) on phencyclidine-induced hypermotility are indirect, as appears from the absence of an effect on locomotor activity 15 minutes after administration of optimum doses of the drugs (Table I), at a time when brain GABA concentrations are still below 3 µmol/g.

The determination of a compound's ability to act as a gamma-aminobutyric acid transaminase inhibitor and its ability to increase brain gaba concentrations by a decrease in GABA-T activity may readily be assessed by standard *in vitro* and *in vivo* assays well known in the art (e.g., ([1]) Jung M.J., Lippert B., Metcalf B.W., Böhlen P., Schechter P.J., 1977: γ-VinylGABA (4-aminohex-5-enoic acid), a new selective irreversible inhibitor of GABA-T: effects on brain GABA metabolism. J. Neurochem. 29: 797-802, and ([2]) Jung M.J., Heydt J.G. and Casara P., 1984: γ-VinylGABA, a new inhibitor of 4-aminobutyrate aminotransferase. Comparison with other inhibitors of this enzyme. Biochem. Pharmacol. 33: 3717-3720) as well as by comparison with successful clinically active gamma-aminobutyric acid transaminase inhibitors (e.g., ([1]) Schechter P.J. and Tranier Y., 1978: The pharmacology of enzyme-activated inhibitors of GABA-transaminase, *in* Enzyme-Activated Irreversible Inhibitors (Seiler, N., Jung, M.J. and Koch-Weser, J., eds.), pp. 149-162, Elsevier/North-Holland Biomedical Press, Amsterdam, and ([2]) Schechter P.J., (1986), Vigabatrin, *in* New Anticonvulsant Drugs (Meldrum, B.S. and Porter, R.J., eds.), pp. 265-275, John Libbey, London).

Based upon the standard techniques, it is to be found that the treatment of phencyclidine intoxication and schizophrenia may be treated by increasing brain gaba concentrations by the administration of an effective amount of a gamma-aminobutyric acid transaminase inhibitor. In practice, the dose of vigabatrin may be in the range of about 1 to 4 grams per day for a 70 kilogram adult, on an oral basis, preferably in two divided doses and about half that dose for (4S)-allenylGABA. Of course, effective dose ranges for other GABA-T inhibitors may readily be determined using similar techniques, particularly in comparison with the foregoing two known GABA-T inhibitors. Of course, the dose range is also dependent upon the nature and severity of the condition, age, general health conditions and such other factors well known and appreciated by the skilled attending diagnostician.

In another aspect, this invention relates to the use of a GABA-T inhibitor, preferably 4-aminohex-5-enoic acid or 4-amino-5,6-heptadienoic acid, either as their racemates or, preferably as the active enantiomer, in the manufacture of a medicament for the treatment of phencyclidine intoxication or in the treatment of schizophrenia.

Another aspect of this invention is the enhancement of the treatment of phencyclidine intoxication and the treatment of schizophrenia with a GABA-T inhibitor by the conjunctive use of glycine, the relative dosages and the modes of administration being analogous to those taught in USP 4,540,582.

The gamma-aminobutyric acid transaminase inhibitors useful for the objects of this invention may be administered either parenterally or enterally using standard techniques well known in the art, preferably using the GABA-T inhibitors in the form of their pharmaceutically acceptable salts.

For oral administration, the compounds can be formulated into solid or liquid preparations such as capsules, pills, tablets, lozenges, melts, powders, suspensions, or emulsions. Solid unit dosage forms can be capsules of the ordinary gelatin type containing, for example, surfactants, lubricants and inert fillers such as lactose, sucrose, and cornstarch or they can be sustained release preparations. In another embodiment, the compounds of Formula I can be tableted with conventional tablet bases such as lactose, sucrose, and cornstarch in combination with binders, such as acacia, cornstarch, or gelatin, disintegrating agents such as potato starch or alginic acid, and a lubricant such as stearic acid or magnesium stearate. Liquid preparations are prepared by dissolving the active ingredient in an aqueous or non-aqueous pharmaceutically acceptable solvent which may also contain suspending agents, sweetening agents, flavoring agents, and preservative agents as are known in the art.

For parenteral administration the compounds may be dissolved in a physiologically acceptable pharmaceutical carrier and administered as either a solution or a suspension. Illustrative of suitable pharmaceutical carriers are water, saline, dextrose solutions, fructose solutions, ethanol, or oils of animal, vegetative, or synthetic origin. The pharmaceutical carrier may also contain preservatives, buffers, etc., as are known in the art.

## Claims

1. The use in the manufacture of a medicament for the treatment of phencyclidine intoxication and for the treatment of schizophrenia of a GABA-T inhibitor.

2. The use in the manufacture of a medicament for the treatment of phencyclidine intoxication of a compound selected from the group consisting of 4-amino-5-enoic acid or 4-amino-5,6-heptadienoic acid.

3. The use in the manufacture of a medicament for the treatment of schizophrenia of a compound selected from the group consisting of 4-amino-5-enoic acid or 4-amino-5,6-heptadienoic acid.

European Patent Office

# PARTIAL EUROPEAN SEARCH REPORT

which under Rule 45 of the European Patent Convention shall be considered, for the purposes of subsequent proceedings, as the European search report

Application Number

EP 91 40 1036

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 520 028 (J.L. ADAMS)<br>* Column 6, lines 25-32,57-65 * | 1 | A 61 K 31/195 |
| Y | CELLULAR AND MOLECULAR NEUROTOXICOLOGY, 1984, edited by T. Narahashi, pages 191-206, Raven Press, New York, US; J.C. NORRIS et al.: "Phencyclidine and GABA system"<br>* Page 191, paragraph 1; page 195, paragraph 2; page 198, paragraph 3; page 201, paragraph 3; page 202, paragraph 2 * | 1,2 | |
| Y | BIOCHEMICAL PHARMACOLOGY, vol. 33, no. 22, 15th November 1984, pages 3717-3720, Pergamon Press, Ltd, Oxford, GB; M.J. JUNG et al.: "Gamma-allenyl GABA, a new inhibitor of 4-amino butyrate amino transferase. Comparison with other inhibitors of this enzyme"<br>* Page 3717, paragraph 1; page 3718, lines 25,26 and 37-46 * | 1,2 | TECHNICAL FIELDS SEARCHED (Int. Cl.5)<br>A 61 K |

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

The compound name "4-amino-5-enoic acid" (see claims 2 and 3) is nonsensical. It has been taken to mean 4-aminohex-5-enoic acid as mentioned in the description. A search has been made on the basis of this amendment.

| Place of search | Date of completion of the search | Examiner |
|---|---|---|
| THE HAGUE | 12-12-1991 | MAIR J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

## CLAIMS INCURRING FEES

The present European patent application comprised at the time of filing more than ten claims.

☐ All claims fees have been paid within the prescribed time limit. The present European search report has been drawn up for all claims.

☐ Only part of the claims fees have been paid within the prescribed time limit. The present European search report has been drawn up for the first ten claims and for those claims for which claims fees have been paid,

namely claims:

☐ No claims fees have been paid within the prescribed time limit. The present European search report has been drawn up for the first ten claims.

## LACK OF UNITY OF INVENTION

The Search Division considers that the present European patent application does not comply with the requirement of unity of invention and relates to several inventions or groups of inventions,
namely:

see sheet -B-

☐ All further search fees have been paid within the fixed time limit. The present European search report has been drawn up for all claims.

☐ Only part of the further search fees have been paid within the fixed time limit. The present European search report has been drawn up for those parts of the European patent application which relate to the inventions in respect of which search fees have been paid,

namely claims:

☒ None of the further search fees has been paid within the fixed time limit. The present European search report has been drawn up for those parts of the European patent application which relate to the invention first mentioned in the claims,

namely claims: point 1.

## LACK OF UNITY OF INVENTION

The Search Division considers that the present European patent application does not comply with the requirement of unity of invention and relates to several inventions or groups of inventions,
namely:

The application shows non-unity a posteriori since part of the claimed uses in claim 1 is already known.

1. Claims 1(partially), 2: Use of GABA-T inhibitors especially 4-aminohex-5-enoic acid and 4-aminohepta-5,6-dienoic acid for treatment of phencyclidine intoxication.

2. Claims 1(partially), 3: Use of GABA-T inhibitors especially 4-aminohex-5-enoic acid and 4-aminohepta-5,6-dienoic acid for treatment of schizophrenia.